# EUROPÄISCHE PATENTSCHRIFT

(11) **EP 2 736 814 B1**
(45) Veröffentlichungstag und Bekanntmachung des Hinweises auf die Patenterteilung: **12.10.2016**
(21) Anmeldenummer: 12720907.0
(22) Anmeldetag: 16.05.2012
(51) Int. Cl.: B65D 1/09, B65D 81/32, A61M 15/00

(54) **EINWEGAMPULLE ZUM EINSETZEN IN EINEN AEROSOLERZEUGER**
SINGLE USE AMPOULE FOR A DEVICE TO CREATE AEROSOLS
AMPOULE À USAGE UNIQUE POUR UN DISPOSITIF DE PRODUCTION D'AÉROSOLS

(30) Priorität: 26.07.2011 DE 102011079810
(43) Veröffentlichungstag der Anmeldung: 04.06.2014
(73) Patentinhaber: PARI Pharma GmbH, 82319 Starnberg (DE)
(72) Erfinder: GALLEM, Thomas, 81371 München (DE); HETZER, Uwe, 81476 München (DE)
(74) Vertreter: Hoffmann Eitle
(86) Internationale Anmeldenummer: PCT/EP2012/059130
(87) Internationale Veröffentlichungsnummer: WO 2013/013852

(56) Entgegenhaltungen:
- DE-A1- 1 486 515
- DE-A1-102005 038 619
- DE-A1-102007 056 462
- DE-C- 570 040

## Beschreibung

Die vorliegende Erfindung betrifft Ampullen, insbesondere Ampullen mit Schnittstellen. Im Besonderen betrifft die vorliegende Erfindung dabei Ampullen für den Einsatz in Aerosolerzeugern, d. h. in Vorrichtungen, die zur Erzeugung von Aerosolen zur topischen Applikation auf die Haut oder in Körperhöhlen bzw. Öffnungen, wie z. B. Nase, Lunge, Gelenke und Bauchraum, verwendet werden können, um Krankheiten von Mensch und Tier zu diagnostizieren, prophylaktisch vorzubeugen, zu immunisieren und/oder zu therapieren. Im Speziellen betrifft die vorliegende Erfindung dabei Ampullen mit den Merkmalen im Oberbegriff von Anspruch

Derartige Ampullen sind aus der DE 10 2005 038 619 A1 oder DE 10 2007 056 462 A1 bekannt. Diese Ampullen werden in der Regel einstückig in einem sogenannten Blow-Fill-Seal-Verfahren gefertigt und gleichzeitig mit der zu vernebelnden Substanz befüllt. Diese Ampullen werden meist als Einmalartikel verwendet, sogenannte Einwegampullen. Dabei sind hinsichtlich der Gestaltung und Geometrie der Ampulle neben den Anforderungen zur Aufnahme und Festlegung im Aufnahmeabschnitt des Aerosolerzeugers die Anforderungen an diesen Herstellungsprozess zu berücksichtigen.

Gerade im Arzneimittelbereich haben sich jedoch neben den sogenannten Blow-Fill-Seal-Ampullen eine Vielzahl anderer Verpackungsformate etabliert, die oftmals hinsichtlich ihrer Ausgestaltung z. B. dem verwendeten Material entsprechend der enthaltenen Substanz ausgebildet sind. Hierbei können unterschiedliche Materialien oder Fertigungsverfahren zum Einsatz kommen. Beispiele sind undurchsichtiges (farbiges) oder durchsichtiges Glas und/oder Kunststoff (z. B. braunes Glas, Spritzgusskunststoff als Flasche, Blister, Vial, Vessel, Kartusche, Vorratskammer etc. Dabei sollten die Verpackungsformen hygienisch unbedenklich sein, was sowohl für Material als auch für die Herstellung (Reinraum und/oder Sterilverfahren) gilt. Gegebenenfalls kann eine Beschichtung vorgesehen sein. Diese kann neben hygienischen, stabilisierende oder weitere chemische und physikalische Eigenschaften beeinflussen und/oder vorgeben.

Das Problem der eingangs beschriebenen einstückig im Blow-Fill-Seal-Verfahren hergestellten Ampullen des Stands der Technik liegt darin, dass den Anforderungen an den Werkstoff (Material) der Ampulle durch deren Inhalt oder Verwendung aufgrund des Blow-Fill-Seal-Verfahrens nicht immer Genüge getan werden kann (das Material muss Blow-Fill-Seal-fähig sein). Bei Verwendung anderer Materialien ist somit ein neuer Herstellungsprozess notwendig.

Darüber hinaus gibt es Substanzen z. B. Medikamente, welche vor der Verabreichung getrennt aufzubewahrende Bestandteile haben, die erst unmittelbar vor der Verabreichung zusammenzubringen, zu kombinieren bzw. zu mischen sind. Dies ist durch die bisher bekannten Ampullen im Blow-Fill-Seal-Verfahren zum Einsatz in Aerosolerzeugern nicht bzw. nur bedingt realisierbar.

Die Aufgabe der vorliegenden Erfindung beruht folglich darin eine Ampulle, insbesondere eine Einwegampulle der eingangs genannten Art so zu verbessern, dass sie in ihrer Gestaltung im Wesentlichen unabhängig vom Fertigungs- und Befüllverfahren zum Einsatz unterschiedlichster Substanzen insbesondere auch Substanzen, die erst unmittelbar vor der Verneblung aus mehreren Bestandteilen zu mischen, anzusetzen bzw. aufzulösen sind, eingesetzt werden kann.

Diese Aufgabe wird durch eine Ampulle mit den Merkmalen des Anspruchs 1 sowie einem Schnittstellenabschnitt einer solchen Ampulle gemäß Anspruch 14 gelöst. Vorteilhafte Weiterbildungen der vorliegenden Erfindung finden sich in den Unteransprüchen.

Der Grundgedanke der vorliegenden Erfindung liegt in der Trennung der bekannten Ampulle in zwei oder mehr Funktionsbereiche bzw. -teile. Diese Trennung der Ampulle in einen Behälterabschnitt und einen Schnittstellenabschnitt (auch als Adapter zu verstehen) ermöglicht eine vom Befüllvorgang losgelöste Fertigung des Schnittstellenabschnitts (Adapters), was zum einen den Einsatz verschiedenster Fertigungs- und Befüllverfahren zulässt und zum anderen Gestaltungsmöglichkeiten, insbesondere des Schnittstellenabschnitts eröffnet. Auch ermöglicht dies die Ausbildung eines Zwei-Kammer-Systems (oder Mehr-Kammer-Systems) mit unterschiedlichen Bestandteilen der zu vernebelnden Substanz in den jeweiligen Kammern, die unmittelbar vor der Verneblung mischbar (kombinierbar, ansetzbar oder auflösbar) sind und die an die zu vernebelnde Substanz angepasste Werkstoffwahl für den Behälterabschnitt. Dementsprechend umfasst die Ampulle zum Einsetzen in einen Aufnahmeabschnitt eines Aerosolerzeugers, um einen in der Ampulle enthaltene Substanz zu vernebeln, einen Behälterabschnitt und einen Schnittstellenabschnitt. Der Behälterabschnitt definiert eine Kammer, in der zumindest ein Bestandteil der Substanz aufgenommen ist. Es kann jedoch auch die gesamte zu vernebelnde Substanz bereits in der Kammer enthalten sein. Insbesondere ist die Substanz unmittelbar vor der Verneblung vorzugsweise eine flüssige Substanz. Der Schnittstellenabschnitt hingegen ist vorzugsweise zur Aufnahme und Festlegung der Einwegampulle in dem Aufnahmeabschnitt des Aerosolerzeuger ausgestaltet, wie er in der eingangs genannten DE 10 2005 083 619 oder der DE 10 2007 056 462 A1 beschrieben ist. Mit anderen Worten werden vorzugsweise sämtliche zur Adaption bzw. Aufnahme und Festlegung der Ampulle in dem Aufnahmeabschnitt des Aerosolerzeugers durch entsprechende Merkmale und Elemente nur des Schnittstellenabschnitts realisiert. Der Behälterabschnitt hingegen ist lediglich als Verpackungshülle für den Bestandteil der Substanz oder die Substanz, die mit dem Aerosolerzeuger zu vernebeln ist, ausgestaltet. Daher ist der Schnittstellenabschnitt zur Aufnahme und Festlegung der Ampulle in dem Aufnahmeabschnitt des Aerosolerzeugers ausgestaltet. Ferner weist er einen Boden auf, der in einer Richtung, in der die Ampulle in den Aerosolerzeuger einzusetzen ist, aufgestoßen werden kann. Hierbei ist unter einer Richtung, in der die Ampulle in den Aerosolerzeuger einzusetzen ist die Richtung zu verstehen, in der die Ampulle relativ zu dem Aufstoßelement bzw. dem Dorn des Aerosolerzeugers zu bewegen ist, um den Boden aufzustoßen. Es ist beispielsweise denkbar den Aufnahmeabschnitt des Aerosolerzeugers feststehend zu gestalten und die Ampulle gegen das Aufstoßelement bzw. den Dorn in den Aufnahmeabschnitt einzudrücken, wodurch der Boden aufgestoßen wird. Alternativ ist auch, wie in dem oben erwähnten Stand der Technik beschrieben, die Möglichkeit denkbar die Ampulle in einem Deckel des Aerosolerzeugers zu fixieren und durch Aufschrauben oder Anbringen des Deckels an dem Aerosolerzeuger die Ampulle in den Aerosolerzeuger einzusetzen und dabei den Boden aufzustoßen. Beide Varianten sind durch die gewählte Formulierung umfasst. Die vorliegende Erfindung kennzeichnet sich dadurch, dass der Behälterabschnitt und der Schnittstellenabschnitt durch separate Teile gebildet sind und jeweils einen Verbindungsbereich aufweisen, in dem der Behälterabschnitt und der Schnittstellenabschnitt miteinander verbunden oder verbindbar sind. Diesbezüglich ist es denkbar die Verbindung bereits herstellungsseitig zu realisieren, wozu beispielsweise Heißumformverfahren (Schweißverfahren), Klebeverfahren, ein Verschrauben, Verrasten oder anderweitiges Verriegeln der separaten Teile gewählt werden können. Hierbei ist der Verbindungsbereich der Teile insbesondere so gestaltet, dass der Schnittstellenabschnitt mit unterschiedlichen oder gleichen Verfahren mit Behälterabschnitten aus unterschiedlichsten Materialien verbunden werden kann. Denkbare Behälterabschnitte sind Folienblister, blasgeformte Behälter, spritzgegossene Behälter sowie Glasbehälter, aber auch im Blow-Fill-Seal-Verfahren hergestellte Behälter. Bei dem Schnittstellenabschnitt handelt es sich vorzugsweise um ein günstiges Spritzgussteil aus Kunststoff. Alternativ oder zusätzlich ist es auch denkbar den Schnittstellenabschnitt nach dem Befüllen mit der/den Substanz/-en aus dem Behälterabschnitt durch einen Deckel zu verschließen, wozu gleichfalls der Verbindungsbereich des Schnittstellenabschnitts verwendet werden kann. Durch die Trennung von Behälter und Schnittstellenabschnitt wird es möglich je nach den Anforderungen an den Schnittstellenabschnitt bzw. den Behälterabschnitt angepasste optimale Herstellungs- und Befüllverfahren zu wählen, wodurch die Gestaltungsfreiheit wesentlich erhöht wird und verschiedenartigste Kombinationen denkbar sind. Diesbezüglich ist es insbesondere vorteilhaft, wenn der Schnittstellenabschnitt unabhängig vom Behälterabschnitt stets gleich ausgestaltet ist, so dass stets der gleiche Schnittstellenabschnitt für unterschiedliche Behälterabschnitte zum Einsatz kommen kann.

Gemäß einer bevorzugten Ausführungsform ist es denkbar insbesondere dann, wenn die Verbindung von Behälter- und Schnittstellenabschnitt erst durch einen Endverbraucher erfolgt, auch den Behälterabschnitt in der Richtung, in der die Ampulle in den Aerosolerzeuger einzusetzen ist, mit einem aufstoßbaren Boden zu versehen, wobei der Schnittstellenabschnitt auf der seinem Boden entgegengesetzten Seite ein Aufstoßelement, z. B. einen hohlen Dorn zum Öffnen des Bodens des Behälters, aufweist.

Dabei ist es einerseits denkbar das Aufstoßelement so zu gestalten, dass der Boden des Behälters während des Verbindens des Behälterabschnitts und des Schnittstellenabschnitts aufgestoßen wird. Mit anderen Worten wird beispielsweise der Endverbraucher den Behälterabschnitt und den Schnittstellenabschnitt verbinden, z. B. miteinander verschrauben oder verrasten, wobei das Verschrauben mittels eines Gewindes oder eines Bajonettverschlusses verfolgen kann. Während dieses Verbindens findet auch eine Relativbewegung des Behälterabschnitts und des Schnittstellenabschnitts aufeinander zu, d. h. entlang der Längsrichtung der Ampulle in einer Richtung, in der die Ampulle in den Aerosolerzeuger einzusetzen ist, statt, während der das Aufstoßelement, z. B. der Hohldorn, den Boden des Behälterabschnitts durchstößt, ähnlich wie das Aufstoßelement des Aerosolerzeugers den aufstoßbaren Boden des Schnittstellenabschnitts. Bezüglich detaillierter Ausgestaltungen dieser Variante wird auf den eingangs genannten Stand der Technik verwiesen.

Alternativ ist es auch denkbar, dass der Behälterabschnitt und der Schnittstellenabschnitt bevorzugt (beim Hersteller, in der Klinik, beim Arzt oder zu Hause beim Patienten) vorab oder vor der Inhalation unlösbar bzw. untrennbar miteinander verbunden werden, jedoch eine Relativverschiebung in Richtung der Längsachse der Ampulle bzw. der Einsetzrichtung der Ampulle in den Aerosolerzeuger gewährleistet bleibt. In diesem Fall ist das Aufstoßelement so ausgestaltet, dass der Boden des Behälterabschnitts nach dem Verbinden des Behälterabschnitts und des Schnittstellenabschnitts durch die Relativbewegung des Behälterabschnitts und des Schnittstellenabschnitts zueinander aufgestoßen wird. Diese Relativbewegung kann durch Gegeneinanderdrücken der zwei oder mehr Abschnitte realisiert werden oder durch den Eingriff von Zapfen an dem Schnittstellenabschnitt oder dem Behälterabschnitt, die in entsprechende Gewindenuten in dem Behälterabschnitt oder dem Schnittstellenabschnitt greifen, so dass eine Rotation des einen Abschnitts relativ zu dem anderen zur besagten Relativbewegung und zum Aufstoßen des Bodens des Behälterabschnitts führt. Die erwähnten Zapfen können dabei ausgeformt sein als Dorne, Adapter, Formstücke und/oder Passstücke.

Um das besagte Zwei-Kammer-System (oder Mehr-Kammer-System) zu realisieren, ist es besonders bevorzugt, dass auch der Schnittstellenabschnitt eine Kammer bildet und im Bereich des Aufstoßelements eine Öffnung aufweist, um die Kammer des Behälterabschnitts nach dem Öffnen des Bodens des Behälterabschnitts mit der Kammer des Schnittstellenabschnitts zu verbinden. Dies kann beispielsweise durch den erwähnten Hohldorn als Aufstoßelement erzielt werden.

In einer Ausführungsform der Erfindung wird vorzugsweise die Kammer des Schnittstellenabschnitts abgedichtet. Dafür kann die Öffnung des Schnittstellenabschnitts durch ein Siegel verschlossen sein. Beim Einsatz eines derartigen Zwei-Kammer-Systems (oder Mehr-Kammer-Systems) enthält die Kammer des Schnittstellenabschnitts einen Bestandteil der zu vernebelnden Substanz, während der andere Bestandteil in der Kammer des Behälterabschnitts vorhanden ist. Die beiden oder mehreren Bestandteile können dabei Substanzen, Wirkstoffe, aktive Komponenten, Trägerstoffe, Trägerlösungen, Abbauprodukte und dergleichen enthalten, die für eine medizinische, therapeutische, diagnostische, immunisierende und/oder analytische Anwendung benutzt werden. Durch Aufstoßen des Bodens des Behälterabschnitts und Öffnen des Siegels können die beiden Bestandteile miteinander vermischt werden und nach dem Einsetzen der Ampulle in den Aerosolerzeuger vernebelt werden. Dabei können die Bestandteile identische und unterschiedliche Inhaltsstoffe (z. B. Wirkstoffe in Pulverform, in Form eines Lyophilisats und/oder liquider Form, wie Trägerlösung) in den unterschiedlichen Kammern enthalten, wobei z. B. das Mischen einer Trägerlösung mit dem Wirkstoff in Pulverform oder als Lyophilisat die Inhalationslösung bzw. die Suspension zur Verneblung bilden kann. Weiter können auch zwei oder mehr Flüssigkeiten miteinander verbunden, gemischt bzw. angesetzt werden, wie z. B. zwei oder mehr unterschiedliche Substanzen enthaltende Flüssigkeiten.

Vorzugsweise sind der Behälterabschnitt und/oder der Schnittstellenabschnitt einstückig (aus einem Stück) ausgebildet und/oder sind verbunden und/oder bilden eine Einheit beim Anwender (z. B. Hersteller, Arzt, Klinik, medizinisches Personal oder Patient).

Darüber hinaus sind der Behälterabschnitt und der Schnittstellenabschnitt um eine Längsachse rotationssymmetrisch ausgestaltet. Dies kann z. B. die Fertigung erleichtern, oder ein freies Einsetzen ohne Orientierungsvorgaben ermöglichen.

Der Boden des Schnittstellenabschnitts und/oder der Boden des Behälterabschnitts können in einer Ebene senkrecht zur Längsrichtung bzw. zur Richtung in der die Ampulle in den Aerosolerzeuger einzusetzen ist, liegen. Darüber hinaus ist es, wie in der DE 10 2007 056 462 A1 beschrieben, vorteilhaft, dass sowohl der Schnittstellenabschnitt und/oder der Behälterabschnitt einen umlaufenden Kragen aufweisen, der eine Wand des entsprechenden Abschnitts über seinen Boden hinaus in seiner Längsrichtung, d. h. in der Richtung in der die Ampulle in den Aerosolerzeuger einzusetzen ist, verlängert, um den Boden und die den Boden umgebende gegebenenfalls vorhandene Sollbruchstelle entsprechend zu schützen. Dabei kann der Kragen des Behälterabschnitts vorzugsweise durch den Verbindungsbereich realisiert werden. Gleichzeitig kann der Kragen die Funktion einer Dichtfläche des Behälterabschnitts gegenüber dem Schnittstellenabschnitt bzw. des Schnittstellenabschnitts gegenüber dem Aerosolerzeuger erfüllen.

Auch ist es bevorzugt, dass der Schnittstellenabschnitt eine umlaufende, nach außen offene Nut in einer Wand zur Festlegung der Ampulle in dem Aufnahmeabschnitt des Aerosolerzeugers, z. B. einem Deckel des Aerosolerzeugers, umfasst. Bezüglich spezieller Ausgestaltungen dieser Art wird ebenfalls auf den eingangs genannten Stand der Technik verwiesen.

Neben der Ampulle (den Ampullen und/oder den Ampullenkammern) schlägt die vorliegende Erfindung auch einen Schnittstellenabschnitt einer solchen bzw. für eine solche Ampulle vor, der sämtliche zur Zusammenwirkung mit dem Aerosolerzeuger notwendigen Komponenten und einen Verbindungsbereich aufweist, um zur Bildung der Ampulle mit einem Behälterabschnitt, wie er oben beschrieben wurde, verbunden zu werden.

Bevorzugterweise ist der Verbindungsbereich dabei als Gewinde und/oder nach Art eines Bajonettverschlusses ausgestaltet. Ebenfalls kann der Verbindungsbereich (bzw. die Koppelung), z. B. durch eine Verrastung, Verriegelung, Verschraubung, Luer-Lock oder dergleichen ausgeführt sein. Alternativ sind auch Schnapp-, Rast-, Klemm-, Pass-, Dicht-, Kleb-, und/oder Haftverbindungen denkbar.

Die separate Fertigung des Schnittstellenabschnitts erweitert den Gestaltungsspielraum derartiger Ampullen und gewährleistet eine genaue und reproduzierbare Fertigung aller für die Verbindung mit dem Aerosolerzeuger funktionsrelevanten Komponenten, insbesondere im Boden des Schnittstellenabschnitts und dem gegebenenfalls vorgesehenen Kragen des Schnittstellenabschnitts, der auch zur Abdichtung gegenüber dem Aufstoßelement z. B. dem Hohldorn des Aerosolerzeugers dienen kann.

Eine Vielzahl der daraus resultierenden Kombinationsmöglichkeiten des Schnittstellenabschnitts mit verschiedenartigen Behälterabschnitten aus unterschiedlichen Materialien und mit unterschiedlicher Gestaltung ermöglicht die Auswahl eines auf den Verpackungs- und Befüllprozess zugeschnittenen Verfahrens zur Herstellung und Befüllung des Behälterabschnitts.

Die Ampulle kann offen oder geschlossen ausgeführt sein. Auch kann der Schnittstellenabschnitt leer sein und im Gebrauch aus dem Behälterabschnitt gefüllt werden. Der Schnittstellenabschnitt kann alternativ oder zusätzlich nach dem Befüllen aus dem Behälterabschnitt mit einem lösbaren oder einem unlösbaren Deckel verschlossen werden.

Ebenfalls ist eine Membran als Verschluss (Abschluss) der Ampulle bzw. des Behälterabschnitts und/oder des Schnittstellenabschnitts denkbar. Durch diese Membran kann, z. B. mithilfe einer Spritze, ein erneutes Befüllen der Ampulle bzw. des Behälterabschnitts und/oder des Schnittstellenabschnitts ermöglicht werden.

Auch kann der Schnittstellenabschnitt zur Bildung der Ampulle mit einer oder mehreren Behälterabschnitten verbunden werden. Dabei kann die Verbindung (bzw. Koppelung) lösbar oder unlösbar erfolgen. Diese Verbindung des Schnittstellenabschnitts zur Bildung der Ampulle, kann dazu dienen diese gas- und/oder flüssigkeitsdicht zu verschleißen.

Eine unlösbare Verbindung wird für die Umsetzung einer Einwegampulle eingesetzt, um z. B. ein erneutes Befüllen zu verhindern. Dies kann als sicherer Verschluss (bzw. Koppelung), z. B. durch eine Verrastung, Verriegelung, Verschraubung, Gewinde, Luer-Lock, Bajonettverschluss oder dergleichen, ausgeführt sein.

Die verschiedenen oder gleichen Verbindungsbereiche von Behälter- und/oder Schnittstellenabschnitt können dabei aus verschiedenen oder gleichen Materialen hergestellt sein. In Frage kommen weiche und harte Kunststoffe. Auch sind Beschichtungen denkbar, welche die Verbindungseigenschaften unterstützen bzw. verbessern.

Darüber hinaus ermöglicht die Trennung in zwei oder mehr Funktionsbereiche bzw. zwei oder mehr Teile die Realisierung einer Zwei- oder Mehr-Kammer-Verpackung, wie es oben erläutert wurde.

Weitere Merkmale und Vorteile der vorliegenden Erfindung, die alleinstehend oder in Kombination mit einem oder mehreren der vorstehenden Merkmale umgesetzt werden können, insofern sich die Merkmale nicht widersprechen, ist aus der folgenden Beschreibung bevorzugter Ausführungsformen ersichtlich. Diese erfolgt unter Bezugnahme auf die begleitenden Zeichnungen, in denen:
Figur 1 eine Ampulle gemäß der vorliegenden Erfindung im Längsschnitt zeigt, wobei der Behälterabschnitt und der Schnittstellenabschnitt nicht verbunden sind;
Figur 2 die Ampulle aus Figur 1 im verbundenen Zustand von Behälterabschnitt und Schnittstellenabschnitt zeigt;
Figur 3a und b eine Ampulle gemäß zwei Ausführungsformen im verbundenen Zustand von Behälterabschnitt und Schnittstellenabschnitt zeigt;
Figur 4 eine Ampulle gemäß einer weiteren Ausführungsform zeigt, wobei der Behälterabschnitt und der Schnittstellenabschnitt nicht verbunden sind;
Figur 5 die Ampulle aus Figur 4, in Figur 5a im verbundenen Zustand von Behälterabschnitt und Schnittstellenabschnitt und in Figur 5b mit durch einen Deckel verschlossenen Schnittstellenabschnitt, zeigt;
Figur 6 eine Ampulle gemäß einer weiteren Ausführungsform mit einem Behälterabschnitt, der zwei Kammern aufweist, in a) einem Zustand, in dem die Behälterabschnitte nicht mit dem Schnittstellenabschnitt in Fluidverbindung stehen und b) einem Zustand, in dem die Kammern des Behälterabschnitts mit dem Schnittstellenabschnitt in Fluidverbindung stehen, zeigen;
Figur 7 eine weitere Ausführungsform einer erfindungsgemäßen Ampulle in den Figuren 7a bis c in einem Bewegungsablauf, in dem der Schnittstellenabschnitt Zug um Zug in die Kammer des Behälterabschnitts eingeschoben wird, zeigt; und
Figur 8a und b weitere Ausführungsformen einer erfindungsgemäßen Ampulle zeigt, wobei der Behälterabschnitt teilweise durch einen Beutel gebildet ist.

Es versteht sich, dass in den begleitenden Zeichnungen gleiche oder vergleichbare Elemente durch die gleichen Bezugsziffern gekennzeichnet sind und in den verschiedenartigen Ausführungsformen auf eine erneute Beschreibung dieser gleichen oder vergleichbaren Elemente zur Vermeidung von Wiederholungen verzichtet wird.

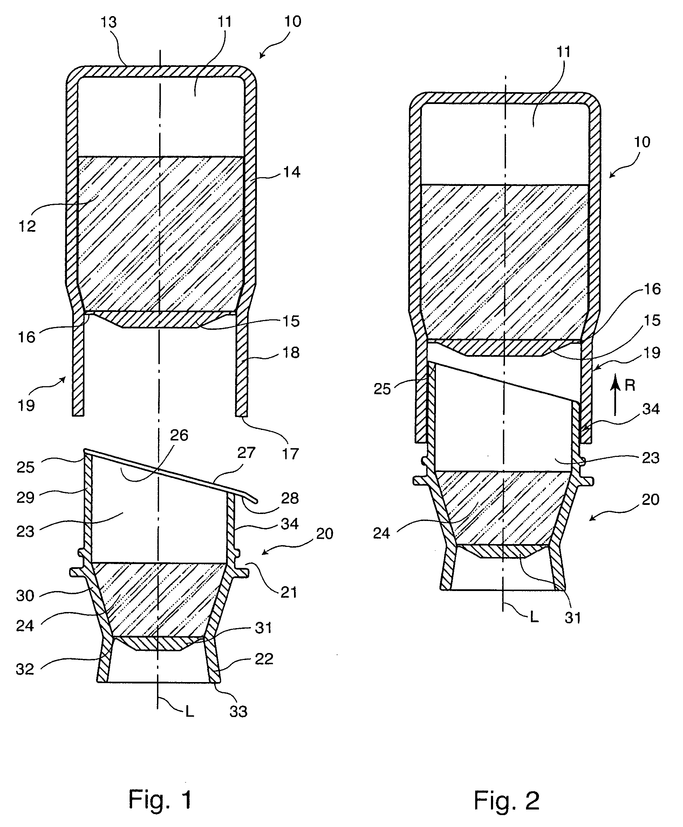

Die in den Figuren 1 und 2 dargestellte Einwegampulle ist eine Zwei-Kammer-Ampulle. Sie umfasst einen Behälterabschnitt 10 und einen Schnittstellenabschnitt 20.

Der Behälterabschnitt 10 weist bei den dargestellten Ausführungsformen eine zylindrische Grundform auf. Er ist um seine Längsmittelachse L im Wesentlichen rotationssymmetrisch. Der Behälterabschnitt 10 bildet eine Kammer 11 (erste Kammer), die einen ersten Bestandteil 12 einer Substanz, die in einem nicht dargestellten Aerosolerzeuger zu vernebeln ist, enthält. Die Kammer 11 wird an einem dem Schnittstellenabschnitt 20 entgegensetzten Ende des Behälterabschnitts 10 durch eine im Wesentlichen senkrecht zur Längsachse L verlaufende Stirnwand 13, die durch den Zylindermantel gebildete Wand 14 sowie einen ebenfalls senkrecht zur Längsachse L verlaufenden Boden 15, der zumindest entlang eines Teilumfangs von einer Sollbruchstelle 16 umgeben ist, geschlossen. Der Boden 15 liegt in einem Abstand von der Stirnseite 17 des Behälterabschnitts 10 entgegen der Endwand (Deckfläche) 13. Um dies zu erzielen, wird die Wand 14 über den Behälterboden 15 hinaus von der Endwand 13 ausgehend in Richtung der Längsachse L verlängert und bildet einen Kragen 18. Der Kragen ist als Verbindungsabschnitt 19 zur Verbindung mit dem Schnittstellenabschnitt 20 ausgestaltet.

Der Schnittstellenabschnitt 20 umfasst die konstruktiven Elemente zur Verbindung bzw. Aufnahme und Festlegung mit dem nicht dargestellten Aerosolerzeuger. Er umfasst eine umlaufende Nut 21 sowie einen Kragen 22, wie sie als konstruktive Elemente in der DE 10 2007 056 462 A1 zur Aufnahme und Festlegung in dem dort beschriebenen Aerosolerzeuger notwendig sind. Ferner definiert der Schnittstellenabschnitt 20 eine weitere Kammer 23 (zweite Kammer), die einen zweiten Bestandteil der zu vernebelnden Substanz, z. B. pulverförmigen Wirkstoff eines Medikaments enthält. An seinem in Längsrichtung L dem Behälterabschnitt 10 zugewandten Ende ist ein Aufstoßelement 25 in Form eines Hohldorns ausgebildet, der eine Öffnung 26 beschreibt, die mit der Kammer 23 in Fluidverbindung steht bzw. mit dieser kommuniziert. Diese Öffnung ist bei der dargestellten Ausführungsform durch ein Siegel 27 fest verschlossen. Das Siegel 27 umfasst eine Grifflasche 28, um das Siegel 27 von der Öffnung 26 zu entfernen und die Kammer 23 frei zu geben. Die Kammer 23 wird durch das Siegel 27 sowie eine Zylinderwandung 29 im oberen Bereich des Schnittstellenelements 20 sowie einem kegelstumpfförmig zulaufenden Kanal 30, der sich an dem dem Behälterabschnitt 10 abgewandten Ende von der Zylinderwandung 29 fortsetzt, begrenzt. Eine weitere Begrenzung erfolgt durch den Boden 31 mit der zumindest teilweise entlang des Umfangs umlaufenden Sollbruchstelle 32. Der Boden 31 liegt gleichfalls senkrecht zur Längsachse L. Der Kragen 22 erstreckt sich von dem der Zylinderwandung 29 abgewandten Ende des kegelstumpfförmigen Abschnitts 30 in Richtung der Längsachse von der Zylinderwand 29 weg über den Boden 31 in Längsrichtung L hinaus, um den Kragen 22 zu bilden, so dass der Boden von der Stirnseite 33 in einem Abstand angeordnet ist und so wie er in der DE 10 2007 056 462 A1 beschrieben ist, geschützt liegt. Die Zylinderwandung 29 bildet gleichzeitig den Verbindungsbereich 34 zum Verbinden mit dem Verbindungsbereich 10 des Behälterabschnitts 10.

Bei einer Ausführungsform ist es denkbar, dass der Behälterabschnitt 10 und der Schnittstellenabschnitt 20 separat zusammen ausgeliefert werden und der Endverbraucher diese zur Verwendung in einem Aerosolerzeuger zunächst verbindet. Hierzu greift der Endverbraucher die Grifflasche 28 des Siegels 27, um dieses von dem Aufstoßelement 25 bzw. dem Schnittstellenabschnitt 20 zu lösen. Dadurch wird die Öffnung 26 frei gegeben. Im Anschluss erfolgt, wie in Figur 2 dargestellt, eine Verbindung des Behälterabschnitts 10 und des Schnittstellenabschnitts 20 im Bereich ihrer Verbindungsbereiche 19 bzw. 34. Dies kann beispielsweise durch eine Schraubverbindung realisiert werden, wobei der Schnittstellenabschnitt 20 ein Außengewinde und der Behälterabschnitt 11 ein Innengewinde aufweisen kann. Diese Ausgestaltung kann jedoch auch umgekehrt gewählt werden. Auch ist eine Schnappverbindung oder ein Bajonettverschluss denkbar. In Figur 2 ist der Schnittstellenabschnitt 20 nicht vollständig in den Behälterabschnitt 10 eingeschraubt, so dass das Aufstoßelement 25 in Form des Hohldorns kurz vor der Sollbruchstelle 26 des Bodens 15 liegt. Durch eine weitere Verschraubung des Schnittstellenabschnitts 20 auf dem Behälterabschnitt 10 erfolgt eine weitere Relativbewegung entlang des Pfeils R, so dass das Aufstoßelement 25 ähnlich wie in Bezug auf den Boden 31 in der DE 10 2007 056 462 A1 beschrieben aufgestoßen und der Boden 15 zur Seite geklappt wird. Dadurch kann z. B. die Trägerlösung 12 als Bestandteil der Substanz von der Kammer 11 im Behälterabschnitt 10 in die Kammer 23 des Schnittstellenabschnitts 20 strömen und mischt sich dort mit dem pulverförmigen Wirkstoff 24 als zweitem Bestandteil der zu vernebelnden Substanz.

Im Anschluss kann die so vorbereitete Ampulle, wie es in der DE 10 2007 056 462 A1 beschrieben ist, in einen Aerosolerzeuger eingesetzt werden, wobei der Boden 31 entlang der Sollbruchstelle 32 aufgestoßen wird und die gemischte Substanz in der Ampulle zum Aerosolerzeuger, z. B. einer vibrierbaren Membran mit einer Vielzahl von Löchern, wie es gleichfalls aus dem erwähnten Stand der Technik bekannt ist, strömt und durch diese hindurch durch Vibration vernebelt wird. Die Vielzahl der Löcher z. B. im Mikrometern Bereich (wie 1 µm bis 6 µm) in der zu vibrierenden Membran, können mit Hilfe von Laser (z. B. Laser Drilling) hergestellt werden.

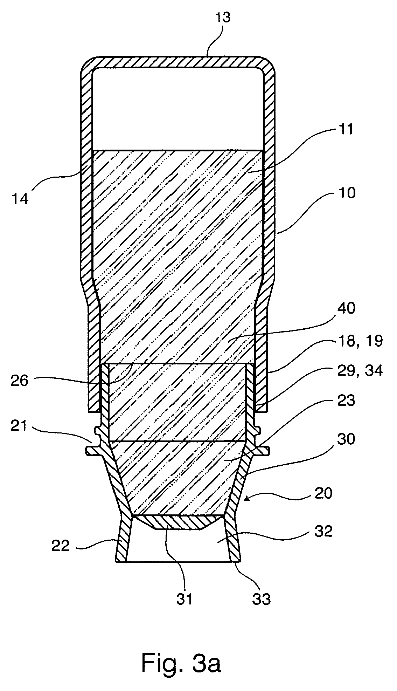

Eine weitere Ausführungsform ist in Figur 3a dargestellt. Diese unterscheidet sich von der Ausgestaltung in den Figuren 1 und 2 primär dadurch, dass die Verbindung des Behälterabschnitts 10 mit dem Schnittstellenabschnitt 20 herstellerseitig erfolgt. Dementsprechend weist der Schnittstellenabschnitt 20 kein Aufstoßelement 25 auf und der Behälterabschnitt 10 keinen Boden 15. Die Substanz 40 ist dabei in der Kammer 11 sowie der Kammer 23 des Behälterabschnitts 10 und des Schnittstellenabschnitts 20 aufgenommen. Die Verbindung des Behälterabschnitts 10 mit dem Schnittstellenabschnitt 20 im Verbindungsbereich 19 bzw. 34 kann durch beliebige Heißumformverfahren z. B. Schweißverfahren, Klebeverfahren, aber auch durch die erwähnten Schraub-, Rast- oder Bajonettverbinden erfolgen.

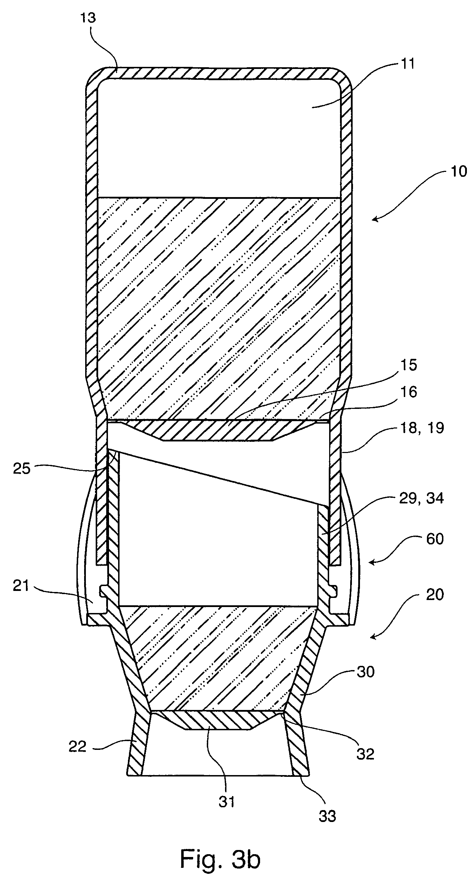

Eine herstellerseitige Verbindung von Behälterabschnitt 10 und Schnittstellenabschnitt 20 ist Figur 3b zu entnehmen, die in dieser Ausgestaltung der Ausführungsform in den Figuren 1 und 2 näher kommt. Durch die bereits herstellerseitige Verbindung von Schnittstellenabschnitt 20 und Behälterabschnitt 10 ist es denkbar durch einen optionalen Hygienering 60 die relativ zueinander beweglichen Verbindungsbereiche 19 und 34 durch einen umlaufenden Hygienering 60 zu schützen und so zu verhindern, dass vor der Verwendung der Ampulle Verunreinigungen von außen in den Schnittstellenbereich und damit in die Substanz gelangen können.

Ansonsten unterscheiden sich diese Ausführungsformen nicht wesentlich von den in den Figuren 1 und 2 dargestellten.

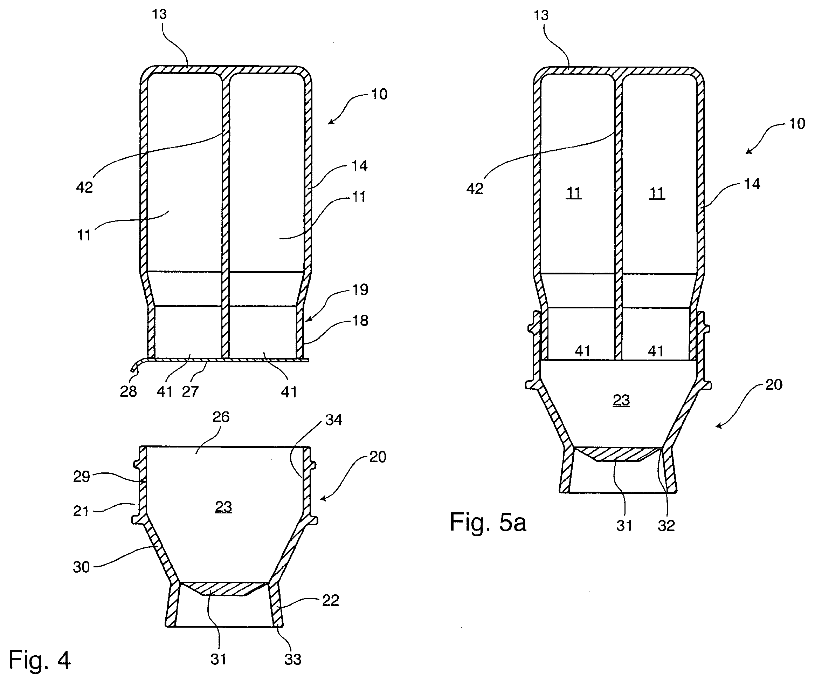

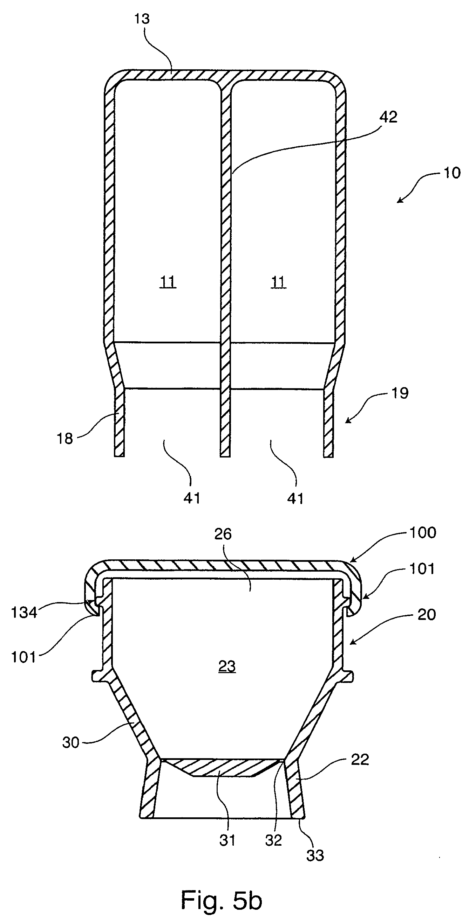

Eine weitere Ausgestaltung ist in den Figuren 4 und 5 dargestellt. Die Ampulle in Figur 4 setzt sich ebenfalls aus einem Behälterabschnitt 10 und einem Schnittstellenabschnitt 20 zusammen. Dabei ist der Schnittstellenabschnitt 20 im Wesentlichen identisch mit dem Schnittstellenabschnitt 20 der Figur 1 ausgestaltet und gleiche oder vergleichbare Elemente werden an dieser Stelle nicht erneut beschrieben. Vielmehr wird auf die Ausführungen zu Figur 1 verwiesen. Anders als bei der Ausgestaltung in Figur 1 umfasst der Schnittstellenabschnitt 20 jedoch keine Schneidkante 25 und ist auch nicht durch eine Versiegelung 27 verschlossen. Vielmehr liegt der Hohlraum 23 über die Öffnung 26 im Wesentlichen frei und es ist auch kein Bestandteil der zu vernebelnden Substanz 40 im Schnittstellenabschnitt 20 aufgenommen. Darüber hinaus weist die dem Behälterabschnitt 10 zugewandte zylindrische Kontur 29 des Schnittstellenabschnitts 20 in dem Verbindungsbereich 34 einen größeren Innendurchmesser als den Außendurchmesser der zylindrischen Wand 18 im Verbindungsbereich 18 des Behälterabschnitts 10 auf.

Auch hier ist der Behälterabschnitt 10 im Wesentlichen zylindrisch. Er ist um seine Längsachse L im Wesentlichen rotationssymmetrisch. Anders als bei der Ausführung in Figur 1 sind jedoch zwei Kammern 11 in dem Behälterabschnitt 10 ausgebildet, die jeweils einen Bestandteil einer Substanz, die in einem nicht dargestellten Aerosolerzeuger zu vernebeln ist, enthalten. Auch ist anders als in der Ausgestaltung in Figur 1 statt des Bodens 15 mit der Sollbruchstelle 16 die der Stirnwand 13 entgegengesetzte Seite des Behälterabschnitts 10 mit jeweiligen Öffnungen 41 zu den Kammern 11 durch ein Siegel 27 verschlossen. Das Siegel 27 umfasst eine Grifflasche 28, um das Siegel 27 von den Öffnungen 41 zu entfernen und die Kammern 11 frei zu geben. Die Kammern 11 werden durch das Siegel 27 sowie die Zylinderwandungen 14, die Stirnwand 13 und eine Trennwand 42 definiert.

Vor dem Gebrauch der Ampulle ist das Siegel 27 zu entfernen, indem die Grifflasche 28 gegriffen und das Siegel 27 von den Öffnungen 41 abgezogen wird. Im Anschluss erfolgt die Verbindung des Schnittstellenabschnitts 20 mit dem Behälterabschnitt 10 über die Verbindungsbereiche 19 bzw. 34. Dies kann wie bei der Ausführungsform in Figur 1 und 2 durch eine Schraubverbindung realisiert werden. Bevorzugt sind jedoch Verbindungen, die ein Lösen des Behälterabschnitts 10 und des Schnittstellenabschnitts 20 nach der Verbindung und damit nach dem Gebrauch der Ampulle nicht mehr ermöglichen, um eine im klinischen Betrieb unerwünschte Weiterverwendung der Komponenten zu vermeiden. Der verbundene Zustand ist in Figur 5a gezeigt. Alternativ zum verbundenen Zustand ist es, wie es in Figur 5b gezeigt ist, auch denkbar nach dem Befüllen der Kammer 23 des Schnittstellenabschnitts 20 aus den Kammern 11 des Behälterabschnitts 10 die Öffnung 26 des Schnittstellenabschnitts 20 durch einen vorzugsweise nach dem Anbringen nicht lösbaren Deckel 100 zu verschließen. Dies kann beispielsweise derart erfolgen, dass der Verbindungsbereich 134 des Schnittstellenabschnitts 20 einen oder mehrerer Vorsprünge 134 (bzw. Aussparungen) aufweist, die im verschlossenen Zustand mit einem oder mehreren Rasthaken 101 des Deckels 100 in Eingriff kommen. Diese Rastverbindung kann unlösbar gestaltet sein, so dass der Deckel 100 nach dem Aufbringen nicht mehr gelöst werden kann. Alternativ könnten die Rasthaken auch am Schnittstellenabschnitt und die Vorsprünge bzw. Aussparungen am Deckel angebracht sein. Im Gebrauch wird, wie oben erläutert, zunächst das Siegel 27 vom Behälter 10 entfernt und die Substanzen aus den Kammern 11, gegebenenfalls durch Verbinden des Behälterabschnitts 10 mit dem Schnittstellenabschnitt 20, in die Kammer 23 ein- und dort zusammengebracht. Im Anschluss wird der Behälterabschnitt 10 gegebenenfalls wieder vom Schnittstellenabschnitt 20 gelöst und der Deckel 100 durch Verrasten auf dem Schnittstellenabschnitt 20 aufgebracht.

Im Anschluss können sich die in den Kammern 11 enthaltenen Bestandteile der Substanz im Hohlraum bzw. der Kammer 23 des Schnittstellenabschnitts 20 zur Bildung der Substanz 40 vermischen bzw. verbinden, bevor die Ampulle beispielsweise, wie es in der DE 10 2007 056 462 A1 beschrieben ist, in einen Aerosolerzeuger eingesetzt wird um die Substanz zu vernebeln.

Ansonsten unterscheidet sich diese Ausführungsform im Wesentlichen nicht von der in Bezug auf die Figuren 1 und 2 beschriebenen Ausgestaltung.

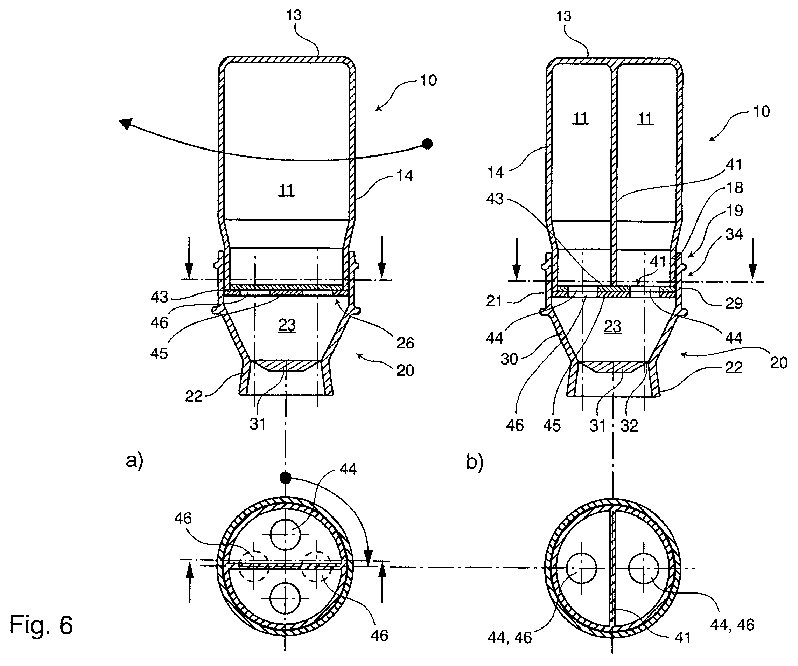

Eine weitere Ausgestaltung ist in Figur 6 gezeigt, wobei auch hier, ähnlich wie in Bezug auf Figur 3 erläutert, eine herstellerseitige Verbindung von Behälterabschnitt 10 und Schnittstellenabschnitt 20 erfolgt. Die Ausgestaltung des Behälterabschnitts 10 und des Schnittstellenabschnitts 20 ist im Wesentlichen identisch zu den Darstellungen in den Figuren 4 und 5, so dass auf die dortigen Ausführungen Bezug genommen wird, um Wiederholungen zu vermeiden.

Anders als in den vorherigen Ausführungsformen erfolgt die Verbindung zwischen dem Behälterabschnitt 10 und dem Schnittstellenabschnitt 20 bei dieser Ausgestaltung derart, dass die Elemente relativ zueinander drehbar sind, wie es durch den Pfeil in den Querschnittsansichten in Figuren 6a und b dargestellt ist.

Die Kammern 11 sind im Bereich ihrer Öffnungen 41 durch einen Boden 43 verschlossen, welcher bei der dargestellten Ausgestaltung zwei Öffnungen 44 aufweist.

Auch die Kammer 23 des Schnittstellenabschnitts 20 ist durch eine Deckelplatte 45 auf der Seite der dem Boden 31 abgewandten Öffnung 26 verschlossen. Auch die Deckelplatte weist zwei Öffnungen 46 auf.

Vor dem Gebrauch der Ampulle werden der Behälterabschnitt 10 und der Schnittstellenabschnitt 20, wie es durch den Pfeil in Figur 6a unten angedeutet ist, relativ zueinander verdreht. Dadurch können die Öffnungen 44 des Bodens 43 und die Öffnungen 46 der Deckelplatte 45 in Deckung gebracht werden, wie es in Figur 6b dargestellt ist. Dadurch wird ermöglicht die Kammern 11 jeweils über die Öffnungen 44 und 46 in Fluidverbindung mit der Kammer 23 zu bringen. Somit ist es denkbar Bestandteile der Substanz aus den Kammern 11 in der Kammer 23 zusammen zu bringen. Auch ist es denkbar zusätzlich in der Kammer 23 einen Bestandteil der Substanz vorzusehen, so dass drei unterschiedliche Bestandteile zusammengebracht werden können. Auch ist durch Anordnung der Öffnungen in Deckelplatte 45 bzw. Boden 43 eine Vermischung der einzelnen Bestandteile in einer speziellen Reihenfolge möglich und/oder über den Öffnungsquerschnitt eine Geschwindigkeit der Vermengung bestimmbar. Auch ist es selbstverständlich möglich, statt lediglich zweier Kammern, drei, vier oder mehr Kammern in einem Behälterabschnitt 10 vorzusehen oder aber mehrere Behälterabschnitte 10 mit jeweils wenigstens einer Kammer mit nur einem Schnittstellenabschnitt 20 zu verbinden.

Im Anschluss erfolgt wiederum das Einsetzen der Ampulle in einen Aerosolerzeuger, wie es oben bereits in Bezug auf die DE 10 2007 056 462 A1 beschrieben wurde.

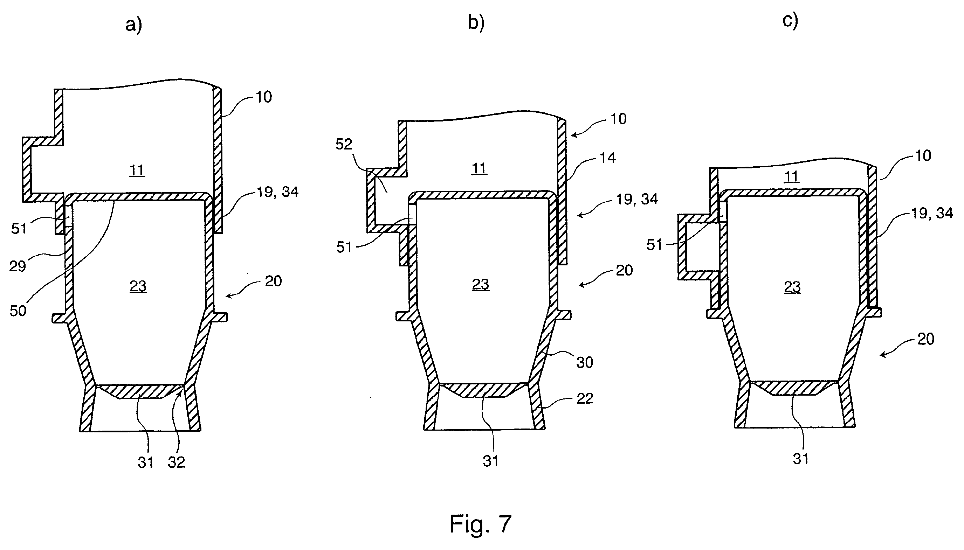

Eine weitere Ausgestaltung ist in Figur 7 erläutert. Ähnlich wie in Figur 6 erfolgt auch bei der Ausführungsform in Figur 7 eine Relativdrehung des Behälterabschnitts 10 und des Schnittstellenabschnitts 20 zueinander, um Bestandteile der Substanz hier in den Kammern 11 und 23 vor Gebrauch zusammen zu bringen. Gleichzeitig erfolgt bei dieser Ausführungsform jedoch auch eine translatorische Bewegung. Hierfür können beispielsweise die Verbindungsabschnitte 19 und 34 in Form einer Schraubverbindung gestaltet sein. Erfolgt eine herstellerseitige Verbindung, ist die Schraubverbindung vorzugsweise derart gestaltet oder eine zusätzliche Einrichtung vorgesehen, dass ein vollständiges Trennen von Behälterabschnitt 10 und Schnittstellenabschnitt 20 verhindert ist. Auch ist es jedoch denkbar, dass die Verbindung erst beim Anwender erfolgt. Hier könnte zunächst ein Rasthaken zu überwinden sein, wenn der Schnittstellenabschnitt 20 in den Behälterabschnitt eingeschraubt wird, um ein späteres Trennen zu vermeiden.

Anders als in den Ausführungsformen zuvor ist die dem Boden 31 entgegengesetzte Seite des Schnittstellenabschnitts 20 durch eine Stirnwand 50 verschlossen und in der zylindrischen Seitenwand 29 ist eine Öffnung 51 vorgesehen. Diese ist im Ausgangszustand in Figur 7a durch den zylindrischen Abschnitt 14 der Wand des Behälterabschnitts 10 überdeckt und damit verschlossen.

Vor dem Gebrauch erfolgt eine Rotation des Schnittstellenabschnitts 20 relativ zum Behälterabschnitt 10 und damit eine translatorische Bewegung des Schnittstellenabschnitts 20 in die Kammer 11 des Behälterabschnitts 10. Dadurch gelangt, wie es in Figur 7b dargestellt ist, die Öffnung 51 über einen Bypass bzw. eine Nebenkammer 52 mit der Kammer 11 in Fluidverbindung, so dass gegebenenfalls Bestandteile in den Kammern 11 und 23 zusammengebracht werden können oder aber eine Substanz, die in der Kammer 11 enthalten ist, in eine leere Kammer 23 einströmt, bevor die Verbindung mit dem Aerosolerzeuger erfolgt. Durch eine weitere Rotation und damit verbundene translatorische Bewegung ist es denkbar nach dem Füllen der Kammer 23 die Öffnung 51 wieder zu verschließen, wie es in Figur 7c dargestellt ist. Dies würde es gegebenenfalls ermöglichen in einem Behälterabschnitt 10 mehrere Dosen aufzunehmen und durch ein Herausschrauben des Schnittstellenabschnitts 20 ein erneutes Befüllen über die Öffnung 51 und die Nebenkammer 52 zu gewährleisten. Es ist jedoch ebenso denkbar, dass die Fluidverbindung der Kammern 11 und 23 über die Öffnung 51 und den Nebenkanal 52 auch im vollständig eingeschraubten Zustand bestehen bleibt.

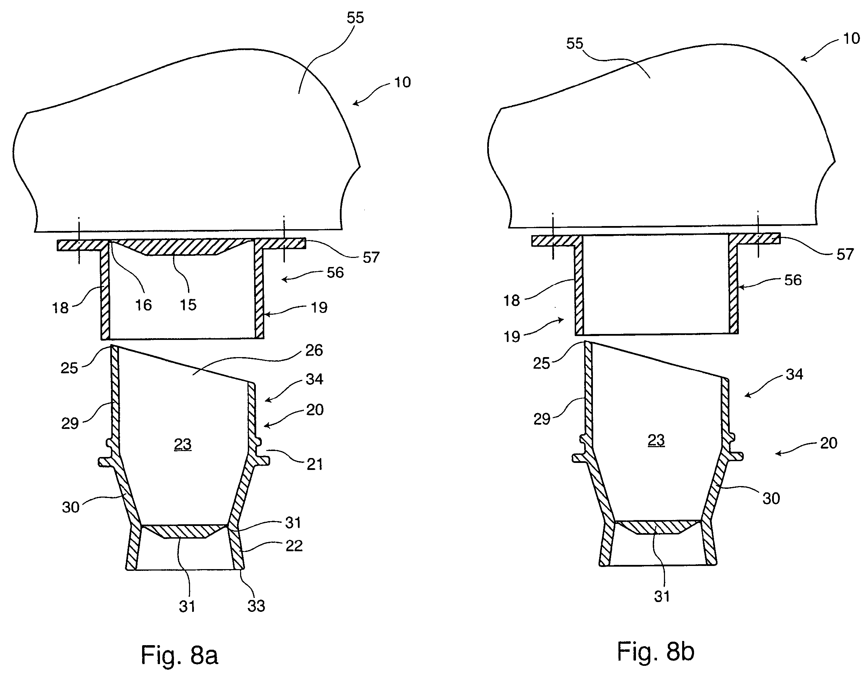

Schließlich ist eine weitere Ausgestaltung der Erfindung in den Figuren 8a und b dargestellt. Bei dieser entspricht der Schnittstellenabschnitt 20 im Wesentlichen dem Schnittstellenabschnitt 20 in den Figuren 1 und 2, wobei jedoch auf das Siegel 27 verzichtet wurde und in der Kammer 23 keine Substanz 24 enthalten ist.

Der wesentliche Unterschied liegt darin, dass statt des in den vorherigen Ausführungsformen beschriebenen zylindrischen und aus Kunststoff bestehenden Behälterabschnitts 10 ein die Kammer 11 enthaltener teilweise dargestellter Beutel 55 zum Einsatz kommt, der mit einem Zwischenstück 56 aus Kunststoff verbunden ist. Dieses Zwischenstück 56 weist einen zylindrischen Verbindungsbereich 19 mit einer Zylinderwand 18 auf, der mit dem Beutel 15 in an und für sich bekannten Verfahren verbunden werden kann. Dies kann beispielsweise durch Heißpräge- oder Siegelverfahren mittels des umlaufenden Flansches 57 erfolgen. Dieser Verbindungsbereich 19 wirkt mit dem Verbindungsbereich 34 zur Verbindung des Behälterabschnitts 10 mit dem Schnittstellenabschnitt 20 zusammen, wie es oben beschrieben wurde.

Beim Verbinden wird der Boden 15 entlang der Sollbruchstelle 16 durch die Schneidkante aufgestochen und eine Fluidverbindung zwischen der Kammer 23 und der im Beutel 55 in Figur 8 nicht sichtbaren Kammer 11 hergestellt, bevor die so gebildete Ampulle, wie zuvor beschrieben, in einen Aerosolerzeuger eingesetzt wird. Alternativ ist es auch, wie in Figur 8b dargestellt, denkbar auf den Boden 15 zu verzichten, wobei der Beutel 55 rundum verschlossen ist. Um die Fluidverbindung herzustellen, wird mittels der Schneidkante 25 beim Verbinden über die Verbindungsabschnitte 19 und 34 ein Abschnitt des Beutels 55 ausgestochen und somit die Fluidverbindung zwischen der Kammer 11 in dem Beutel 55 und der Kammer 23 hergestellt.

Es versteht sich, dass die vorliegend Erfindung nicht auf die obigen Ausführungen begrenzt ist, sondern dass verschiedenartige Modifikationen und Abwandlungen vorgenommen können, wobei die Ansprüche den Schutbereich festlegen. Darüber hinaus ist die vorliegend Erfindung nicht auf die Verwendung einer speziellen Substanz oder spezielle Bestandteile der Substanz beschränkt.

## Patentansprüche

1. Ampulle zum Einsetzen in einen Aerosolerzeuger, um eine in der Ampulle enthaltene Substanz (40) zu vernebeln, umfassend:
einen Behälterabschnitt (10), der eine Kammer (11, 23) zur Aufnahme zumindest eines Bestandteils der Substanz (40) bildet; und
einen Schnittstellenabschnitt (20), der zur Aufnahme und Festlegung der Ampulle in dem Aufnahmeabschnitt des Aerosolerzeugers ausgestaltet ist und in einer Richtung, in der die Ampulle in den Aerosolerzeuger einzusetzen ist, einen aufstoßbaren Boden (15, 31) aufweist,
**dadurch gekennzeichnet, dass**
der Behälterabschnitt (10) und der Schnittstellenabschnitt (20) durch separate Teile gebildet sind,
der Schnittstellenabschnitt verschließbar ausgestaltet ist, und
der Behälterabschnitt (10) und der Schnittstellenabschnitt (20) um eine Längsachse rotationssymmetrisch ausgestaltet sind.

2. Ampulle nach Anspruch 1, bei der der Behälterabschnitt (10) und der Schnittstellenabschnitt (20) jeweils einen Verbindungsbereich (19, 34) aufweisen, über den der Behälterabschnitt (10) und der Schnittstellenabschnitt (20) miteinander verbunden oder verbindbar sind, wodurch der Schnittstellenabschnitt verschlossen bzw. verschließbar ist.

3. Ampulle nach Anspruch 1 oder 2, bei der auch der Behälterabschnitt (10). in der Richtung, in der die Ampulle in den Aerosolerzeuger einzusetzen ist, einen aufstoßbaren Boden (15, 31) aufweist, und der Schnittstellenabschnitt (20) auf der seinem Boden (15, 31) entgegengesetzten Seite ein Aufstoßelement (25) zum Öffnen des Bodens (15, 31) des Behälters aufweist.

4. Ampulle nach Anspruch 3, bei der das Aufstoßelement (25) so ausgestaltet ist, dass der Boden (15, 31) des Behälterabschnitts (10) während des Verbindens des Behälterabschnitts (10) und des Schnittstellenabschnitts (20) aufgestoßen wird.

5. Ampulle nach Anspruch 3, bei der der Behälterabschnitt (10) und der Schnittstellenabschnitt (20) nach ihrer Verbindung relativ zueinander in der Richtung, in der die Ampulle in den Aerosolerzeuger einzusetzen ist, verschiebbar sind und das Aufstoßelement (25) so ausgestaltet ist, dass der Boden (15, 31) des Behälterabschnitts (10) nach dem Verbinden des Behälterabschnitts (10) und des Schnittstellehabschnitts (20) durch die Relativbewegung des Behälterabschnitts (10) und des Schnittstellenabschnitts (20) zueinander aufgestoßen wird.

6. Ampulle nach einem der Ansprüche 3 bis 5, bei der der Schnittstellenabschnitt (20) eine weitere Kammer (11, 23) bildet und im Bereich des Aufstoßelements (25) eine Öffnung (26) aufweist, um die Kammer (11, 23) des Behälterabschnitts (10) nach dem Öffnen des Bodens (15, 31) des Behälterabschnitts (10) mit der Kammer (11, 23) des Schnittstellenabschnitts (20) zu verbinden.

7. Ampulle nach Anspruch 6, bei der die Öffnung (26) des Schnittstellenabschnitts (20) durch ein Siegel (27) verschlossen ist, die Kammer (11, 23) des Schnittstellenabschnitts (20) einen weiteren Bestandteil der Substanz (40) enthält und die Bestandteile der Substanz (40) in der Kammer (11, 23) des Behälterabschnitts (10) und der Kammer (11, 23) des Schnittstellenabschnitts (20) nach dem Öffnen des Siegels (27) und des Bodens (15, 31) des Behälterabschnitts (10) mischbar sind.

8. Ampulle nach einem der vorstehenden Ansprüche, bei der der Behälterabschnitt (10) und der Schnittstellenabschnitt (20) jeweils einstückig ausgebildet sind.

9. Ampulle nach einem der vorstehenden Ansprüche, bei der der Boden (15, 31) des Schnittstellenabschnitts (20) und der Boden (15, 31) des Behälterabschnitts (10) in einer Ebene senkrecht zu der Richtung, in der die Ampulle in den Aerosolerzeuger einzusetzen ist, liegen.

10. Ampulle nach einem der vorstehenden Ansprüche, bei der der Schnittstellenabschnitt (20) und der Behälterabschnitt (10) einen umlaufenden Kragen (18) aufweisen, der entsprechend eine Wand (14) des Schnittstellenabschnitts (20) oder des Behälterabschnitts (10) über seinen Boden (15, 31) hinaus in der Richtung in der die Ampulle in den Aerosolerzeuger einzusetzen ist verlängert.

11. Ampulle nach einem der vorstehenden Ansprüche, bei der der Schnittstellenabschnitt (20) eine umlaufende, nach außen offene Nut (21) in einer Wand (14) zur Festlegung der Ampulle in einem Aufnahmeabschnitt des Aerosolerzeugers aufweist.

12. Ampulle nach einem der vorstehenden Ansprüche, bei der der Schnittstellenabschnitt (20) und/oder der Behälterabschnitt (10) ganz oder teilweise aus weichem oder hartem Kunststoff in Blow-Fill-Seal-Technik, Spritzgusstechnik und/oder aus Glas gefertigt sind.

13. Ampulle nach einem der vorstehenden Ansprüche, bei der der Behälterabschnitt (10) und/oder der Schnittstellenabschnitt (20) als Einwegartikel ausgestaltet sind, die nach der Anwendung nicht wieder verwendbar sind.

14. Schnittstellenabschnitt (20) für eine Ampulle nach einem der vorstehenden Ansprüche, wobei
der Schnittstellenabschnitt (20) zur Aufnahme und Festlegung der Ampulle in dem Aufnahmeabschnitt des Aerosolerzeugers ausgestaltet ist,
der Schnittstellenabschnitt (20) in einer Richtung, in der die Ampulle in den Aerosolerzeuger einzusetzen ist, einen aufstoßbaren Boden (15, 31) aufweist,
der Schnittstellenabschnitt (20) verschließbar ausgestaltet ist, und
der Schnittstellenabschnitt (20) um eine Längsachse rotationssymmetrisch ausgestaltet ist.

15. Schnittstellenabschnitt (20) nach Anspruch 14, bei dem der Schnittstellenabschnitt (20) einen Verbindungsbereich (34) zur Verbindung mit einem Behälterabschnitt (10) oder einem Deckel aufweist, der bevorzugt als Gewinde, Bajonettverschluss oder Teil einer Rastverbindung ausgestaltet ist.

16. Schnittstellenabschnitt (20) nach Anspruch 15, der nur einmal mit dem Deckel oder dem Behälterabschnitt verschließbar ist, indem der Verbindungsbereich so gestaltet ist, dass nach dem Verbinden ein zerstörungsfreies Lösen des Behälterabschnitts oder Deckels vom Schnittstellenabschnitt (20) verhindert wird.

17. Schnittstellenabschnitt (20) nach Anspruch 15, der einen Verbindungsbereich aufweist, der als zu öffnender Kanal ausgestaltet ist.

18. Schnittstellenabschnitt (20) nach Anspruch 14 bis 17, der zur Bildung der Ampulle mit zwei oder mehr Behälterabschnitten verbindbar bzw. kombinierbar ist.

19. Schnittstellenabschnitt (20) nach Anspruch 14 bis 18, der als Einwegartikel für die Zuführung einer oder mehrerer Medikamentensubstanzen zu einem Aerosolerzeuger zur Aerosolgenerierung, der z. B. bis zu einem Monat, bevorzugt bis zu 14 Tagen und am meisten bevorzugt bis zu maximal 7 Tagen in dem Beatmungskreislauf eines Beatmungsgeräts zur Anwendung kommt.

20. Schnittstellenabschnitt (20) nach einem der Ansprüche 14 bis 19, der einen Verbindungsabschnitt zum Verbinden mit dem Aerosolgenerator aufweist, wobei der Aerosolgenerator ein Membranvernebler ist.

## Claims

1. Ampoule for insertion in an aerosol generator for nebulising a substance (40) contained in the ampoule, comprising:
a receptacle section (10) which forms a chamber (11, 23) for receiving at least one component of the substance (40); and
an interface section (20) which is designed to receive and fix the ampoule in the receiving section of the aerosol generator and has a bottom (15, 31) which can be pushed open in a direction in which the ampoule is to be inserted in the aerosol generator,
**characterised in that**
the receptacle section (10) and the interface section (20) are formed by separate parts,
the interface section is designed so as to be closable, and
the receptacle section (10) and the interface section (20) are designed so as to be rotationally symmetrical about a longitudinal axis.

2. Ampoule according to claim 1, in which the receptacle section (10) and the interface section (20) in each case have a connecting region (19, 34) by means of which the receptacle section (10) and the interface section (20) are or can be connected to each other, as a result of which the interface section is closed or closable.

3. Ampoule according to claim 1 or 2, in which the receptacle section (10) has a bottom (15, 31) which can be pushed open in the direction in which the ampoule is to be inserted in the aerosol generator, and the interface section (20) has a push-open element (25) for opening the bottom (15, 31) of the receptacle on the side opposite its bottom (15, 31).

4. Ampoule according to claim 3, in which the push-open element (25) is designed in such a way that the bottom (15, 31) of the receptacle section (10) is pushed open during connection of the receptacle section (10) and the interface section (20).

5. Ampoule according to claim 3, in which the receptacle section (10) and the interface section (20) are, after connection, slidable relative to each other in the direction in which the ampoule is to be inserted in the aerosol generator, and the push-open element (25) is designed in such a way that, after connection of the receptacle section (10) and the interface section (20), the bottom (15, 31) of the receptacle section (10) is pushed open by the movement of the receptacle section (10) and the interface section (20) relative to each other.

6. Ampoule according to any of claims 3 to 5, in which the interface section (20) forms a further chamber (11, 23) and has an opening (26) in the region of the push-open element (25) for connecting the chamber (11, 23) of the receptacle section (10), after opening of the bottom (15, 31) of the receptacle section (10), to the chamber (11, 23) of the interface section (20).

7. Ampoule according to claim 6, in which the opening (26) of the interface section (20) is closed by a seal (27), the chamber (11, 23) of the interface section (20) contains a further component of the substance (40), and the components of the substance (40) can be mixed in the chamber (11, 23) of the receptacle section (10) and the chamber (11, 23) of the interface section (20) after opening of the seal (27) and the bottom (15, 31) of the receptacle section (10).

8. Ampoule according to any of the preceding claims, in which the receptacle section (10) and the interface section (20) are in each case constructed in one piece.

9. Ampoule according to any of the preceding claims, in which the bottom (15, 31) of the interface section (20) and the bottom (15, 31) of the receptacle section (10) are located in a plane perpendicularly to the direction in which the ampoule is to be inserted in the aerosol generator.

10. Ampoule according to any of the preceding claims, in which the interface section (20) and the receptacle section (10) have a peripheral collar (28) which accordingly lengthens a wall (14) of the interface section (20) or of the receptacle section (10) beyond its bottom (15, 31) in the direction in which the ampoule is to be inserted in the aerosol generator.

11. Ampoule according to any of the preceding claims, in which the interface section (20) has a peripheral, outwardly opening groove (21) in a wall for fixing the ampoule in a receiving section of the aerosol generator.

12. Ampoule according to any of the preceding claims, in which the interface section (20) and/or the receptacle section (10) are partly or completely manufactured from soft or hard plastic using blow-fill-seal technology or injection moulding technology and/or from glass.

13. Ampoule according to any of the preceding claims, in which the receptacle section (10) and/or the interface section (20) are designed as disposable articles which are not re-usable after use.

14. Interface section (20) for an ampoule according to any of the preceding claims, wherein
the interface section (20) is designed to receive and fix the ampoule in the receiving section of the aerosol generator,
the interface section (20) has a bottom (15, 31) which can be pushed open in a direction in which the ampoule is to be inserted in the aerosol generator,
the interface section (20) is designed so as to be closable, and
the interface section (20) is designed so as to be rotationally symmetrical about a longitudinal axis.

15. Interface section (20) according to claim 14, in which the interface section (20) has a connecting region (34) for connection to a receptacle section (10) or a cover which is preferably designed as a screw thread, bayonet fastening or part of a latch connection.

16. Interface section (20) according to claim 15, which is only once closable with the cover or the receptacle section, by designing the connecting region in such a way that, after connection, non-destructive release of the receptacle section or cover from the interface section (20) is prevented.

17. Interface section (20) according to claim 15, which has a connecting region which is designed as a channel to be opened.

18. Interface section (20) according to claims 14 to 17, which can be connected to or combined with two or more receptacle sections to form the ampoule.

19. Interface section (20) according to claims 14 to 18, which is used as a disposable article for the delivery of one or more drug substances to an aerosol generator for aerosol generation which is used e.g. up to one month, preferably up to 14 days and most preferably up to not more than 7 days in the ventilation circuit of a ventilator.

20. Interface section (20) according to any of claims 14 to 19, which has a connecting section for connection to the aerosol generator, wherein the aerosol generator is a membrane nebuliser.

## Revendications

1. Ampoule pour l'utilisation dans un dispositif de production d'aérosol, pour nébuliser une substance (40) contenue dans l'ampoule, comprenant :
une section de contenant (10) qui forme une chambre (11, 23) pour la réception d'au moins un composant de la substance (40) ; et
une section d'interface (20) qui est conçue pour la réception et la fixation de l'ampoule dans la section de réception du dispositif de production d'aérosol et qui présente, dans une direction dans laquelle l'ampoule doit être utilisée dans le dispositif de production d'aérosol, un fond (15, 31) pouvant être actionné,
**caractérisée en ce que**
la section de contenant (10) et la section d'interface (20) sont formées par des parties séparées,
la section d'interface est conçue de façon à pouvoir être fermée, et
la section de contenant (10) et la section d'interface (20) sont conçues de façon symétrique en rotation autour d'un axe longitudinal.

2. Ampoule selon la revendication 1, où la section de contenant (10) et la section d'interface (20) présentent respectivement une zone de liaison (19, 34) par laquelle la section de contenant (10) et la section d'interface (20) sont reliées ou peuvent être reliées, moyennant quoi la section d'interface est ou peut être fermée.

3. Ampoule selon la revendication 1 ou 2, où également la section de contenant (10) présente dans la direction dans laquelle l'ampoule doit être utilisée dans le dispositif de production d'aérosol, un fond (15, 31) pouvant être actionné, et la section d'interface (20) présente sur le côté opposé à son fond (15, 31) un élément d'actionnement (25) pour ouvrir le fond (15, 31) du contenant.

4. Ampoule selon la revendication 3, où l'élément d'actionnement (25) est conçu de telle sorte que le fond (15, 31) de la section de contenant (10) est actionné pendant la liaison de la section de contenant (10) et de la section d'interface (20).

5. Ampoule selon la revendication 3, où la section de contenant (10) et la section d'interface (20) peuvent être déplacées après leur liaison l'une par rapport à l'autre dans la direction dans laquelle l'ampoule doit être utilisée dans le dispositif de production d'aérosol et l'élément d'actionnement (25) est conçu de telle sorte que le fond (15, 31) de la section de contenant (10) est actionné après la liaison de la section du contenant (10) et de la section d'interface (20) par le mouvement relatif de la section de contenant (10) et de la section d'interface (20) l'une par rapport à l'autre.

6. Ampoule selon l'une des revendications 3 à 5, où la section d'interface (20) forme une autre chambre (11, 23) et dans la zone de l'élément d'actionnement (25) présente une ouverture (26) pour relier la chambre (11, 23) de la section de contenant (10) après l'ouverture du fond (15, 31) de la section de contenant (10) avec la chambre (11, 23) de la section d'interface (20).

7. Ampoule selon la revendication 6, où l'ouverture (26) de la section d'interface (20) est fermée par un joint d'étanchéité (27), la chambre (11, 23) de la section d'interface (20) contient un autre composant de la substance (40) et les composants de la substance (40) dans la chambre (11, 23) de la section de contenant (10) et de la chambre (11, 23) de la section d'interface (20) sont miscibles après l'ouverture du joint d'étanchéité (27) et du fond (15, 31) de la section de contenant (10).

8. Ampoule selon l'une des revendications précédentes, où la section de contenant (10) et la section d'interface (20) sont conçues respectivement d'un seul tenant.

9. Ampoule selon l'une des revendications précédentes, où le fond (15, 31) de la section d'interface (20) et le fond (15, 31) de la section de contenant (10) sont dans un plan perpendiculaire par rapport à la direction dans laquelle l'ampoule doit être utilisée dans le dispositif de production d'aérosol.

10. Ampoule selon l'une des revendications précédentes, où la section d'interface (20) et la section de contenant (10) présentent un collet périphérique (18) qui prolonge de façon correspondante une paroi (14) de la section d'interface (20) ou de la section de contenant (10) au-dessus de son fond (15, 31) dans la direction dans laquelle l'ampoule doit être utilisée dans le dispositif de production d'aérosol.

11. Ampoule selon l'une des revendications précédentes, où la section d'interface (20) présente une rainure (21) périphérique ouverte vers l'extérieur dans une paroi (14) pour fixer l'ampoule dans une section de réception du dispositif de production d'aérosol.

12. Ampoule selon l'une des revendications précédentes, où la section d'interface (20) et/ou la section de contenant (10) sont faites complètement ou partiellement d'une matière plastique souple ou dure dans une technique de formage-remplissage-scellage, une technique de moulage par injection et/ou sont en verre.

13. Ampoule selon l'une des revendications précédentes, où la section de contenant (10) et/ou la section d'interface (20) sont conçues comme des articles à usage unique qui ne sont plus réutilisables après l'utilisation.

14. Section d'interface (20) pour une ampoule selon l'une des revendications précédentes, dans laquelle
la section d'interface (20) est conçue pour la réception et la fixation de l'ampoule dans la section de réception du dispositif de production d'aérosol,
la section d'interface (20) présente dans une direction dans laquelle l'ampoule doit être utilisée dans le dispositif de production d'aérosol, un fond (15, 31) pouvant être actionné,
la section d'interface (20) est conçue de façon à pouvoir être fermée, et
la section d'interface (20) est conçue de façon symétrique en rotation autour d'un axe longitudinal.

15. Section d'interface (20) selon la revendication 14, où la section d'interface (20) présente une zone de liaison (34) pour la liaison avec une section de contenant (10) ou un couvercle qui est configurée de préférence comme un filetage, une fermeture à baïonnette ou une partie d'une liaison par encliquetage.

16. Section d'interface (20) selon la revendication 15, qui ne peut être fermée qu'une seule fois par le couvercle ou la section de contenant par le fait que la zone de liaison est conçue de telle sorte qu'après la liaison, un détachement sans destruction de la section de contenant ou du couvercle par rapport à la section d'interface (20) est empêché.

17. Section d'interface (20) selon la revendication 15, qui présente une zone de liaison qui est conçue comme un canal à ouvrir.

18. Section d'interface (20) selon la revendication 14 à 17, qui peut être reliée ou combinée pour la formation de l'ampoule avec deux sections de contenant ou plus.

19. Section d'interface (20) selon la revendication 14 à 18 qui est conçue comme article à usage unique pour l'acheminement d'une ou plusieurs substances médicamenteuses dans un dispositif de production d'aérosol pour la génération d'un aérosol qui est utilisé par exemple jusqu'à un mois, de préférence jusqu'à 14 jours et de manière préférée entre toutes, jusqu'à au maximum 7 jours dans le circuit respiratoire d'un appareil de ventilation.

20. Section d'interface (20) selon l'une des revendications 14 à 19, qui présente une section de liaison pour assurer la liaison avec le générateur d'aérosol, dans laquelle le générateur d'aérosol est un nébuliseur à membrane.
